# EUROPEAN PATENT APPLICATION

(11) **EP 1 335 204 A2**
(43) Date of publication of application: **13.08.2003**
(21) Application number: 03250756.8
(22) Date of filing: 06.02.2003
(51) Int. Cl.: G01N 33/543, G01N 33/58

(54) **Methods for making microbar encoders for bioprobes**

(30) Priority: 06.02.2002 US 72837
(71) Applicant: Agilent Technologies, Inc., Palo Alto, CA 94306 (US)
(72) Inventor: Roitman, Daniel B., Menlo Park, CA 94025 (US); Seaward, Karen L., Palo Alto, CA 94306 (US)
(74) Representative: Tollett, Ian

(57) **Abstract**

A method of making a plurality of substantially identical microbar encoders (40), the microbar encoders having a characteristic detectable signal and capable of linking to a probe molecule. In these methods, one or more layers (15, 16, 17) are sequentially deposited unsupported by a template onto a substrate (10), each layer comprising a plurality of indicator materials. The deposited layers are divided into the plurality of microbar encoders (30a-30d). Diverse groups of microbar encoders can be made separately, and these diverse members can be mixed to provide an anisotropic array for screening multiple target molecules in a massively parallel manner. The present inventive methods thus result in larger scale, efficient production of distinguishable encoders.

## Description

The present invention relates to a method of making a plurality of microbar encoders and an assembly of the same. In particular it relates to assemblies of distinguishable microbar encoders that are used as molecular tags in the detection, identification and/or quantification of target particles, including the detection, identification and/or quantification of biological molecules.

The development of microarrays, microfluidics and miniaturized biosensors has enabled massively parallel screening, in which a sample can be simultaneously screened for thousands of different molecules, such as proteins, nucleic acid sequences, antibodies, etc. In a microarray, such as a genechip, thousands of different DNA fragments are immobilized on a defined surface. The unique sequence of the immobilized fragment at each coordinate of the array is known, so that when a sample is introduced to the array and hybridization is found to occur at specific array coordinates, the identity of gene fragments within the sample can be deduced from the coordinates (and hence, the complementary immobilized fragments) where they are bound. This is known as spatial multiplexing because the target molecule is identified based on the spatially defined position of the probe molecule.

However, there are various issues associated with high throughput applications using arrays. For example, use of a probe molecule in an array requires relatively large sample volumes to cover the whole surface area of the array. In addition, agitation or mixing is required to distribute the sample over the array, which is challenging because the sample is spread in a thin film and not all of the sample material is in contact with the entire surface of the array. Further, there is a lack of reproducibility from array to array, as spotting is not defect-free and no two arrays are identical. Arrays technology also requires large surfaces, which results in very expensive glass handling and array manufacturing.

Various methods and labels have been used to map the identity of the target molecules being screened. This includes the use of identifying tags labeling the probe molecule specifically bound to the target molecule. These identifying tags can be dyes, e.g., an intercalating dye specific for hybridized nucleic acids, or fluorescent labels, e.g., fluorescein, rhodamine, phycoerythrin, and the like. Use of a distinct identifying tag specific for an individual probe molecule to identify the target molecule is known as color or spectral multiplexing.

The applicability of screening multiple samples using color multiplexing was limited previously by the small number of molecules that could feasibly be distinguished during detection, due to the broad emission wavelengths of conventional fluorescent labels and the fact that these labels generally suffer from short-lived fluorescence, i.e., undergo photobleaching after minutes of exposure to an excitation light source. Semiconductor nanocrystals were developed in response to these limitations.

Semiconductor nanocrystals, commonly known as "quantum dots," are specifically capable of absorbing energy from either a particle beam or an electromagnetic radiation source (of broad or narrow bandwidth), and are capable of emitting detectable electromagnetic radiation in a narrow wavelength band when so excited. Quantum dots may be grown in a core/shell configuration wherein a first semiconductor nanocrystal forms a core, and then shells of other semiconductors having controlled thickness of several monolayers are grown surrounding the core. See U.S. Patent No. 6,333,110 (Barbera-Guillem). In addition, quantum dots may be passivated with an inorganic coating, or "shell," uniformly deposited thereon, which can result in an increase in the quantum yield of the fluorescence emission, depending on the nature of the inorganic coating. The particular wavelength band emitted from a particular core/shell quantum dot then can be adjusted according to the both the size and composition of the core and shell layers, and the number of shell layers surrounding the core.

Labeling of the probe molecules with quantum dots thus advantageously permits simultaneous use of a plurality of differently colored quantum dots to be used in a single assay without significant spectral overlap in wavelengths of emitted light. Therefore, each different probe molecule can be labeled with a differently colored quantum dot. Another advantage of the quantum dots lies in processes that involve elevated temperatures. Quantum dots may withstand use at elevated temperatures, including use in processes which comprise thermal cycling steps, i.e., processes which comprise one or more steps in which the temperature is cycled between a low temperature and a high temperature, such as polymerase chain reaction (PCR). The advantage of the high degree of thermal stability of the quantum dots may also be applied to other processes that require elevated temperatures or methods using thermostable organisms or biomolecules derived from thermostable organisms.

Examples of quantum dots known in the art have a core selected from nanocrystals of Group II, VI semiconductors, such as MgS, MgSe, MgTe, CaS, CaSe, CaTe, SrS, SrSe, SrTe, BaS, BaSe, BaTe, ZnS, ZnSe, ZnTe, CdS, CdSe, CdTe, HgS, HgSe, and HgTe as well as mixed compositions thereof, and nanocrystals of Group III, V semiconductors such as GaAs, InGaAs, InP, and InAs and mixed compositions thereof, which nanocrystals are capable of emitting electromagnetic radiation upon excitation. *See* U.S. Patent No. 6,207,392 (Weiss et al.) The use of Group IV semiconductors such as germanium or silicon, and the use of organic semiconductors, may also be feasible under certain conditions. The semiconductor nanocrystals can also include alloys comprising two or more semiconductors selected from the groups described previously, and combinations thereof.

In one approach, different quantities and sizes of identifying tags having characteristic spectral emission signatures, for example, fluorophores such as quantum dots, can be incorporated into polymeric microbeads at precisely controlled ratios. *See* Han et al., Nature Biotechnology, 19(7):621-2 (2001). Their optical properties, e.g., size-tunable emission and simultaneous excitation, render these highly luminescent fluorophores useful for both wavelength-and-intensity multiplexing. The encoded beads are linked to characteristic probe molecules that bind to specific target molecules. The transducing substance or substances, as the case may be, provide detectable identifying tags for the beads. If binding to a bead is detected, the identity of the probe molecule, and hence the target molecule, can be deduced from the identifying tag of the encoded bead. Thus, for example, the bead may include two differently colored quantum dots having a specific intensity ratio.

Since the encoder identifier on the bead effectively performs the function of the known coordinates in the microarray technique, encoded beads eliminate the need for immobilizing the capture or probe molecules used to bind the target molecules in the sample. In addition, the target molecule can be tagged independently of the bead, provided that the tag for the target molecule is distinguishable from the tag encoding the bead, which is useful to indicate that the target molecule is bound to the probe molecule. However, because of the manner by which the encoded beads are fabricated, there is generally a high variability in the amount of quantum dots incorporated per bead, directly translating to decreased accuracy in microbead identification.

Recently, nanobar or microbar codes, which are freestanding, rod-shaped particles having varied segments along the length of the rod, have been developed. See WO 01/25002 (Natan et al.); WO 01/25510 (Natan et al.). Such microbar codes provide another approach in that a small number of distinct physical or chemical indicia, such as electromagnetic, magnetic, optical, spectrometric, spectroscopic or reflectivity indicia, can be arranged in numerous different patterns of various lengths and sizes, and can thus provide for a large pool of uniquely identifiable encoders. Generally, a template or mold is utilized into which the materials that constitute the various segments are introduced. For example, WO 01/25510 discloses electrochemical deposition of metals inside a template with ultrasonic bath and temperature control. This approach emphasizes fabrication of individual, distinguishable encoders, each of which has specific physical or chemical indicia, wherein the shape and size of the encoders is defined by the template within which the encoders are produced. However, use of templates to produce the encoders is impractical to implement with materials such as cross-linked resins, plastics and ceramics and, furthermore, it is difficult to incorporate a quantum dot or other photoluminescent material during such fabrication.

Accordingly, there remains a need for large-scale, efficient manufacture of substantially identical encoders, ultimately resulting in generation of a large numbers of distinguishable encoders, which are fabricated without use of a template or solid support to define the shape of the encoders.

The microbar encoders of the present invention, which have a characteristic detectable signal and are capable of linking to a probe molecule, are made by depositing, unsupported by a template, one or more layers and dividing the layers into the plurality of microbar encoders. The microbar encoders produced according to the present inventive methods have one or more layers, wherein at least one of the layers comprises a transducing material. In some embodiments a plurality of substantially identical microbar encoders are made by laminating together pre-formed layers or by co-extrusion of multiple film layers. Additionally, an assembly of differential microbar encoders of the present invention are made by making at least a first and second plurality of microbar encoders, as described, wherein the first and second plurality of microbar encoders have different characteristic detectable signals. The present inventive methods thus result in large scale, efficient production of distinguishable encoders without the use of a template.

A number of preferred embodiments of the invention will now be described with reference to the drawings.

In the following figures, like reference numerals are used to indicate identical and/or analogous structures shown throughout the figures.

FIGS 1A-D schematically depict a microbar encoder produced according to the methods of the present invention; in FIG 1A, a probe molecule is attached to a top surface layer; in FIG 1B, a probe molecule is attached to a top surface layer and to both lateral surfaces; in FIG 1C, two different probe molecules are attached to a top surface layer and to both lateral surfaces; and in FIG 1D, a probe molecule is also attached to a top surface layer and both lateral surfaces.

FIG 2 schematically illustrates a method of making microbar encoders according to the present invention.

FIG 3 schematically illustrates a method of making microbar encoders according to the present invention in which pre-formed layers are laminated together.

FIG 4 schematically illustrates a method of making microbar encoders according to the present invention in which multiple layers are co-extruded.

The present invention provides various methods for making microbar encoders. Some methods of the present invention involve making a plurality of highly uniform microbar encoders having substantially identical characteristic detectable signals and capable of linking to a probe molecule, wherein the microbar encoders are made without use of a template. The microbar encoders produced according to the present inventive methods have one or more layers, wherein at least one of the layers comprises a transducing material. The combination of transducing material(s) found in the one or more layers of the microbar encoder results in the characteristic detectable signal.

Other methods of the present invention involve making a diverse population of the microbar encoders having different characteristic signals. Such a diverse population, or assembly, of microbar sensors can be made by making a plurality of substantially identical microbar encoders and then making at least one different plurality of substantially identical microbar encoders, such that the different plurality of microbar sensors have different characteristic detectable signals and, therefore, members of the population have different characteristic signals.

The indicator or transducing material of the present invention can be any suitable material that is detectable by any chemical or physical means, including electromagnetic, magnetic, optical, spectrometrical, spectroscopic or mechanical means. Preferably, the transducing material produces a detectable signal in response to exposure to energy. A detectable signal, as used herein, is meant to include any emission of energy, including electromagnetic radiation (visible or infrared or ultraviolet light) and thermal emission, and any other signal or change in signal emanating from the transducing material (including diffraction) and/or absorption in response to exposure of the transducing material to energy. Preferably, the detectable signal produced by the transducing material is an electromagnetic emission or absorption.

Suitable transducing materials include various organic dyes, transducing semiconductors, such as quantum dot nanocrystals, inorganic phosphors, metal-organic phosphors, fluorescent dyes, pigments, photoluminescent polymer molecules, scattering powders (TiO₂), absorbing powders (carbon black), three-dimensional photoluminescent dendrimer molecules (branching oligomers built generationally from a central core, characterized by discrete, controllable molecular architectures), and any combination thereof. Metals capable of emitting electromagnetic radiation can also be used, including, for example, silver, gold, copper, nickel, palladium, platinum, cobalt, rhodium and iridium. Also useful in the context of the present invention are metal-organic compounds capable of emitting electromagnetic radiation, such as aluminum tris (8-hydroxyquinoline) and those described in U.S. Patent No. 6,303,238 (Thompson et al.). *See also* Hoshino et al., Appl. Phys. Lett. 69(2), 224-226 (1996); Kido et al., Chemistry Letters 657-660 (1990); Kido et al., J. Alloys and Compounds, 192: 30-33 (1993); Kido et al., Appl. Phys. Lett., 65: 2124-2126 (Oct. 1994); Kido et al., Jpn. J. Appl. Phys., 35: L394-L396 (Mar. 1996); Forrest, Chemical Reviews, 97: 1793-1896 (Sep./Oct. 1997); Tang et al., Appl. Phys. Lett 51: 913 (Sep. 1987); Forrest et al., Laser Focus World, 99-107 (Feb. 1995); Johnson et al., Journal of the American Chemical Society, 105: 1795-1802. (1983); Hosokawa et al., Appl. Phys. Lett., 67(26): 3853-3855 (Dec. 1995); Adachi et al., Jpn. J. Appl. Phys. 27:L269-L271 (Feb. 1988).

Preferably, the transducing or indicator materials are dispersed colloidally, dissolved in solution and/or chemically bonded in a matrix. Any material that allows transduction of the detectable signals is a suitable matrix. Examples of suitable matrix materials include ceramics, semiconductors, glasses, organic polymer materials and hybrid organic/inorganic materials such as siloxanes. Polymeric materials are particularly suitable for producing composite layers because of the many known techniques for dispersing materials in polymeric matrices, and for depositing polymeric materials in multilayer films. It is noted that such polymeric materials include polymeric glass precursors and hybrid materials such as a siloxane.

In the context of the present inventive methods, the inorganic transducing materials are preferably solublized or dispersed in the matrix and/or layers of the microbar encoder. Any method, including those used in biological or molecular detection systems as known by those skilled in the art, can be used to render the transducing material water-soluble, can be used to render the transducing material soluble in the matrix.

The combination of transducing material found in the one or more layers of the microbar encoder results in the characteristic detectable signal. Therefore, one layer can contain, for example, quantum dots that emit red light in a narrow wavelength band in response to exposure to a broad band of energy, a second layer can contain quantum dots that emit yellow light in response to the same exposure and a third layer can contain quantum dots that emit blue light in response to the same exposure. The characteristic detectable signal produced by the exemplary microbar encoder will be Red-Yellow-Blue. It should be appreciated by one of skill in the art that the present inventive methods can be utilized to produce any combination of layers of transducing materials. Moreover, any number of layers can be thereby produced.

Furthermore, each layer of the microbar encoder can contain one or more transducing materials, each of which produces a detectable signal. For example, one layer can contain quantum dots ("A dots") that emit red light in a narrow wavelength band in response to exposure to a broad band of energy, and second quantum dots ("B dots") that emits yellow light in response to the same exposure. Having such constituent transducers, this layer is then detectable based on the characteristic red and yellow light that is emitted from the layer in response to excitation. Furthermore, the ratio of the number of A dots and B dots can be varied to produce a ratio of intensities of red and yellow light, thus providing a more specific and more easily identifiable characteristic signal.

The microbar encoders of the present invention can be used as identifying tags in virtually any application where quantum dots or other indicators have been used, which different applications are familiar to those of skill in the art. *See, e.g*., U.S. Patent No. 6,326,144 (Bawendi et al.). Furthermore, these microbar encoders of the present invention can be read or detected using any existing instrumentation or software for such indicators, including optical detection mechanisms, scanning probe techniques, electron beam techniques and/or electrical, mechanical or magnetic detection mechanisms, as well as instruments or software specifically designed for the present invention. For example, a flow cytometer device can be used to read the microbars bound to target molecules and the microbar encoders unbound to target molecules; as the microbars traverse the optical readout of the cytometer, the readout can be either limited to recognizing just the wavelengths and intensities associated with the encoded tags, or more desirably, the readout should have sufficient spatial resolution to resolve the relative and absolute positions of the various layers of the microbar.

Generally, the microbar encoder linked to a probe molecule (microbar sensor) of the present invention can be used in technologies such as medical (and non-medical) microscopy, histology, flow cytometry (cell sorting), in-situ hybridization assays (medical assays and research), DNA sequencing, immuno-assays, binding assays, separation, etc. Such assemblies also can be used in applications in the fields of genomics, proteomics and combinatorial chemistry, including multiplexed diagnostics, drug discovery and screening analysis, gene expression analysis, and microorganic monitoring. Genomics involves the qualitative and quantitative measurement of gene activity by detecting and quantitating expression at the messenger RNA level, while proteomics involves the qualitative and quantitative measurement of gene activity by detecting and quantitating expression at the protein level, rather than at the messenger RNA level. Proteomics also involves the study of non-genome encoded events including the post-translational modification of proteins, interactions between proteins, and the location of proteins within the cell. Combinatory chemistry, using small molecule libraries, can involve the concomitant use of affinity chromatography, gene expression profiling and complementation to identify compounds and their protein targets in biological systems. *See, e.g.*, U.S. Patent No. 6,287,864 (Hefti).

The microbar encoders of the present invention are particularly useful for the detection, identification and/or quantification of target molecules or particles, and in particular biological materials, such as, for example, cells and cellular components (deoxyribonucleic acids (DNA) and ribonucleic acids (RNA), proteins, etc.). To facilitate detection, the microbar encoders are linked, either covalently or non-covalently, to a probe molecule specific for the target molecule. The probe molecule must specifically bind to the target molecule, so that the presence of the detectable target material may be subsequently ascertained. The probe molecule can be attached to the microbar encoder directly or through a linking agent. Alternatively, two types of linking agents can be utilized. Furthermore, it is within the contemplation of this invention that the microbar encoder can also be chemically modified after it has been made in order to link effectively to the probe molecule. When linked to a probe molecule, the microbar encoders are referred to as microbar sensors.

The particular probe molecule linked to the microbar encoder of the invention will be selected based on its affinity for the particular target substance whose presence or absence, for example, in a biological material, is to be ascertained. Basically, the probe molecule may comprise any molecule capable of being linked to one or more microbar encoders and that is also capable of specific recognition of a particular target substance. In general, any probe molecule useful in the prior art in combination with a dye molecule to provide specific recognition of a target substance will find utility in the formation of the microbar encoders of the present invention.

Such probe molecules include, for example, biological molecules such as nucleic acids (DNA and RNA), monoclonal and polyclonal antibodies, proteins, polysaccharides, lipids and small molecules such as sugars, peptides, drugs, and ligands. Lists of such probe molecules are available in the published literature, by way of example, in the "Handbook of Fluorescent Probes and Research Chemicals", (sixth edition) by R. P. Haugland, available from Molecular Probes, Inc.

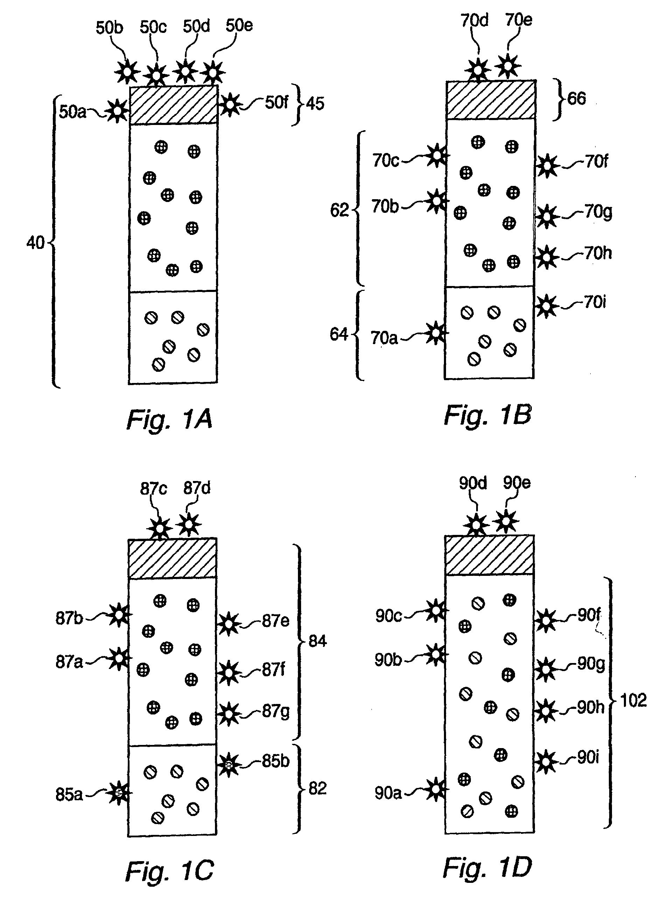

It should be appreciated that one or more probe molecules can be linked to a single microbar encoder. In addition, two or more different probe molecules can be linked to a single microbar encoder. This is demonstrated in FIG 1C, described in detail below. These probe molecules can be linked to the top layer of the microbar encoder (FIG 1A) and/or to the proximal surfaces of the microbar encoders (FIG 1B-D).

A linking agent in the context of the present invention is any substance capable of attaching one or more probe molecules with one or more microbar encoders. By attached is meant, for purposes of the present invention, fused or bound or an association of sufficient stability to withstand conditions encountered in a method of detection between the probe molecule and the microbar encoder. As known to those skilled in the art, and as will be more apparent by the following description, there are several methods and compositions in which the probe molecule and the microbar encoder may be linked, including but are not limited to, bifunctional reagents/molecules, biotin, avidin, free chemical groups (e.g., thiol, or carboxyl, hydroxyl, amino, amine, sulfo, etc.), and reactive chemical groups (reactive with free chemical groups). There is no particular size or content limitations for the linker so long as it can fulfill its purpose of attaching the probe molecule to the microbar encoder.

Linkers are specifically known to those skilled in the art to include, but are not limited to, chemical chains, chemical compounds, carbohydrate chains, peptides, haptens, and the like. Depending on such factors as the molecules to be linked, and the conditions in which the method of detection is performed, the linker may vary in length and composition for optimizing such properties as flexibility, stability, and resistance to certain chemical and/or temperature parameters. For example, short linkers of sufficient flexibility include, but are not limited to, linkers having from 2 to 10 carbon atoms (see, e.g., U.S. Pat. No. 5,817,795 (Gryaznov, et al.)). A variety of references summarize the standard classes of chemistry which may be used to this end, in particular the "Handbook of Fluorescent Probes and Research Chemicals", (6th edition) by R. P. Haugland, available from Molecular Probes, Inc., and the book "Bioconjugate Techniques", by Greg Hermanson, available from Academic Press, New York.

The linkers can also include, but are not limited to, homobifunctional linkers and heterobifunctional linkers. Heterobifunctional linkers, well known to those skilled in the art, contain one end having a first reactive functionality to specifically link a first molecule, and an opposite end having a second reactive functionality to specifically link to a second molecule. As illustrative examples, to operably link a hydroxyl group of a polynucleotide strand to an amino group of a diaminocarboxylic acid, the linker may have: a carboxyl group to form a bond with the polynucleotide, and a carboxyl group to form a bond with the diaminocarboxylic acid (see, e.g., U.S. Pat. Nos. 5,792,786, and 5,780,606 for various linkers known in the art). Heterobifunctional photoreactive linkers (e.g., phenylazides containing a cleavable disulfide bond) are known in the art. For example, a sulfosuccinimidyl-2-(p-azido salicylamido) ethyl-1,3'-dithiopropionate contains a N-hydroxy-succinimidyl group reactive with primary amino groups, and the phenylazide (upon photolysis) reacts with any amino acids.

In another example, oligonucleotide probe molecules that target for specific DNA or RNA sequences can be linked by coating the microbar encoder with a layer of amine-terminated PEG, cross-linking the PEG with 1, 4-phenylene diisothiocyanate and then binding the cross-linker to an amino-modified oligonucleotide.

It should be noted that a plurality of polymerizable linking agents can be used together to form an encapsulating net or linkage around an individual microbar encoder. This is of particular interest where the specific linking agent is incapable of forming a strong bond with the microbar encoder. Examples of linking agents capable of bonding together in such a manner include, but are not limited to: diacetylenes, styrene-butadienes, vinyl acetates, acrylates, acrylamides, vinyl, styryl, and the aforementioned silicon oxide, boron oxide, phosphorus oxide, silicates, borates and phosphates, as well as polymerized forms thereof.

In addition, or as an alternative, to the linking agents, the outside surface of the microbar encoders can be modified using various chemicals or solvents to render the microbar encoders compatible with the probe and to further facilitate linking of the probe to the microbar encoder. Furthermore, the outside surface of the microbar encoders can be modified to facilitate dispersion of the microbar encoders in the various types of buffers used to conduct the assays. In this respect the chemistry of modifying latex beads is applicable (see, e.g., "The Latex course 2001", San Diego, Ca April 30-May 2, 2001, Bangs Laboratories Inc, Fishers, In, and references therein). It is also possible using depositing films of SiO₂, Al₂O₃ TiN, etc, to coat the microbars after fabrication (for example, before cleaving them from the substrate) by well known methods like low temperature plasma enhanced chemical vapor deposition (PECDV), sputtering sol-gel chemistry, etc. Electrostatically driven adsorption is also suitable for coating the microbars with a polyelectrolyte linker or/and charged probe molecule(s) directly on the microbars.

Presence of the detectable target substance within the sample material can be determined by any suitable method that is capable of indicating the binding of the probe molecule to the detectable target molecule, i.e., any method resulting in production of the characteristic detectable signal upon binding of the target molecule to the probe molecule linked to the microbar encoder (microbar sensor). Microbar sensors can be used to determine the presence of target molecules in a sample material by dispersing a plurality of microbar sensors to permit the probe molecules of the sensors to bind to the target molecules in the material. Generally, after introduction of the microbar sensor into the material, unbound microbar sensors are removed from the material, leaving only bound microbar sensors. The sample material (and microbar sensors therein) are then exposed to an energy source capable of causing the microbar sensors to provide their characteristic detectable signals. The presence or absence of the target molecules can then be determined based on which detectable signal is present in the sample material.

Additionally, the target molecules can be labeled or tagged with a different transducing material. Any suitable transducing material discussed previously can be used to label the target molecule, provided that the transducing material used to label the target molecule is readily distinguishable from the transducing material contained in the microbar encoder. Examples of specifically useful transducing materials in this context include DNA intercalating dyes, which indicate the presence of double stranded DNA, and biotin molecules (including native biotin or a biotin derivative having avidin-binding activity; e.g., biotin dimers, biotin multimers, carbo-biotin, and the like) conjugated with a labeled avidin molecule. The presence or absence of the target molecules can then be determined based on whether both, different detectable signals are present in the sample material.

In one embodiment, the microbar sensor (microbar encoder and probe molecule) and the target molecule are both dispersed in a solution. Further, the target molecule is labeled with a transducing agent, as described previously. Determination of the presence of the target bound to the probe molecule of the microbar sensor is based on detection of both of the different, characteristic detectable signals bound to the target and probe molecules. For example, when using a DNA intercalating dye, once the target molecule has bound to the probe molecule linked to the microbar encoder, the characteristic detectable signal can be determined, either by the spatial location or specific pattern, as well as the presence of double stranded DNA, which is present only when the target molecule binds to the probe molecule.

As an alternative to adding the microbar sensors to the sample, the sample may be in a carrier, such as an aqueous solution, that is introduced to the microbar sensors. Here, the target molecules must be tagged with a different, identifying transducing material, which has been described previously. The microbar sensors are, preferably, attached to a solid support in an array. When the microbar sensors are bound to a solid support, the identity of the target molecule(s) within the sample can be determined from the spatial orientation of the array.

Also alternatively, when the unbound microbar sensors have not been removed from the sample material, presence of the bound microbar sensors can be determined (and distinguished from the unbound microbar sensors) by a plurality of methods, including determining the spatial segregation of more intense detectable, characteristic signals arising as a result of the localization of the bound microbar sensors, as opposed to random dispersion (resulting in spatially random characteristic detectable signals) of the unbound microbar sensors. In addition, the target molecules can be grouped according to the type of probe molecule to which the target is bound. The individual target molecules can then be removed from the microbar sensors and each group of target molecule investigated individually. Investigation can include any number of different assays, including conventional DNA, RNA and protein analyses, as well as various chemical analyses, such as mass spectrometry, for example.

Furthermore, the target molecule can be immobilized on a solid support, examples of which include conventional immobilization techniques for nucleotides and proteins, such as immobilization on nitrocellulose filters. The labeled microbar encoders are in a carrier, which is then introduced to the microbar sensors. When the microbar sensors are bound to a solid support, the identity of the target molecule(s) within the sample can be determined from the spatial orientation of the target molecules bound to the solid support.

A suitable carrier, in the context of the present invention, is any type of matter that has little or no reactivity with the microbar sensors, and enables storage and application of the microbar sensors to the sample material. Such materials can be a liquid, including many types of aqueous solutions or biologically derived aqueous solutions (e.g. plasma from blood). Other liquids include alcohols, amines, and any other liquid that neither reacts with nor causes the dissociation of the components of the microbar sensor. The carrier also comprises a substance that will not interfere with the treatment or analysis being carried out by the microbar sensors in connection with the detectable target substance in the sample.

Furthermore, microbar sensors can readily be applied in standard sandwich type immunoassays (ELISA assays), in which a microbar sensor linked to an antibody acts as the stationary phase. When an antigen in a sample is exposed to the microbar sensor and binds to the antibody, a second fluorescently-tagged antibody binds to the antigen and indicates the presence of the bound antigen.

Another illustration of the use of microbar encoders produced according to the present inventive methods is in flow cytometry analysis. Flow cytometry, as used in the prior art, involves contacting a sample material, containing cells, with one or more dyes, or dye conjugated affinity molecules, which are capable of detecting certain molecules or substances on the surface or interior of those cells. Instead of using a dye molecule, a material containing cells may alternatively be labeled with a plurality of microbar sensors. Since each separate microbar sensor is capable of producing a characteristic detectable signal (in response to energy), which is distinguishable from the characteristic detectable signals produced by other microbar sensor that have been exposed to the same energy, the presence of more than one microbar sensor, each bonded to one or more different detectable substances, may be simultaneously detected in a single compartment.

In another illustration of microbar sensors fabricated according to the present invention, different probe molecules can be bound to different segments of the microbar encoder, allowing each microbar sensor to be used to screen for multiple different target molecules. For example, incorporating both a nucleic acid test with an antibody test for a disease can be implemented by linking a microbar encoder having green and blue segments with both oligonucleotide and antibody probe molecules. To facilitate accurate detection, the oligo probe molecules may be linked to the green segments of the microbar encoder, while the antibody probe molecules can be linked to the blue segments of the microbar encoder. Additionally, if the assay is performed as a sandwich, with fluorescently-tagged antibodies and oligo sequences, a positive identification of the disease or biological condition can be made when the entire length of the microbar sensor (i.e., all segments) fluoresce. Detection rates for biological conditions can thus be improved by incorporating a plurality of different tests in a single assay, vastly reducing the probability of false positives that may occur if only a single test is used.

According to the present invention, more than one substantially identical microbar encoders are fabricated. Layers are deposited, unsupported by a template, with each layer including material that can transduce a signal and provide a means to identify the layer. A template, in the context of the present invention, is meant to include any support, be it solid or otherwise, used to define the dimensions of the microbar encoder, which is readily appreciated by one of skill in the art. Once a desired number of layers have been deposited, the resulting layered structure is divided into a number of substantially identical microbar encoders. The microbar encoders are then linked to probe molecules to form microbar sensors, which can, in turn, selectively bind to one or more target molecules present in a sample.

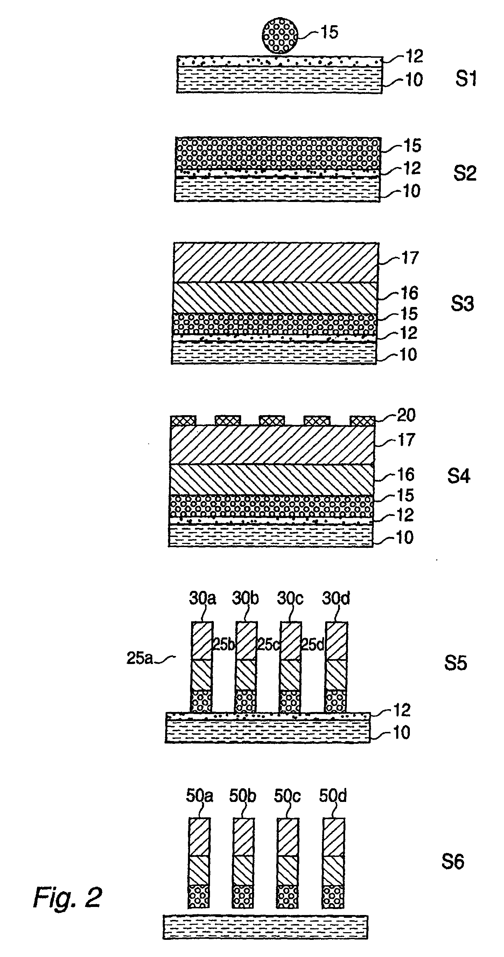

FIG 2 illustrates a first exemplary embodiment of a method of fabricating microbar encoders according to the present invention. It should be noted that, as shown, there is no template used to manufacture the microbar encoders.

Before step S1, during pre-fabrication preparation, materials that have signal transducing properties, or a matrix and a transducing material, are dispersed, liquefied, or converted into a vapor, in a manner that allows these materials to be deposited in thin layers.

In step S1, a first removable layer 12 is deposited onto a substrate 10. The substrate can be rigid; for example, a glass or silicon wafer, or it can be a flexible material, such as a metallic foil or thermoplastic. The removable layer 12 can be any material that can be selectively dissolved, such as aluminum oxide. The removable layer 12 can be patterned with a series of recesses and protuberances using an embossing or lithography technique to produce particular optical effects, such as lasing, anisotropic emissions, interferometric effects, etc.

In step S2, a first layer 15 is deposited over the removable layer 12, unsupported by a template. The first polymeric layer 15 contains one or more transducing materials, each of which transduce a signal in a characteristic manner. In an exemplary embodiment, the first layer 15 comprises a polymer composite, which is spin cast from a liquid solution. In spin casting a thin layer 15 is formed over the removable layer 12 by action of centrifugal forces, which act to spread the polymeric material, creating a highly homogeneous layer. The thickness of the deposited layer 15 (and the further layers deposited of it) can be varied, but typically ranges from 1 to 50 microns. Since the removable layer 12 can be patterned with a series of recesses and protuberances, the first deposited layer 15 will, in conforming to the contour of the removable layer 12, acquire a pattern on its bottom surface. The pattern can be in the form of a grating that can modify optical properties of the layer.

According to the depicted embodiment, in step S3, additional continuous layers of polymeric material 16 and 17 are cast, e.g., spin cast, layer-by layer over the first polymeric layer 15, again, unsupported by a template. It is noted that while three additional layers are shown, this number is merely exemplary, and the number of additional layers can be varied according to the desired complexity of the final microbar encoder. Although additional layers are not required (in which case the first polymeric layer comprises the length of the encoder), the number of additional layers that can be used to define a microbar encoder is unlimited. Each of the additional layers 16 and 17 can be differentiable from one another (and from first polymeric layer 15) based on their length and on the different signal transduction that occurs at each layer. Alternatively, one or more layers 15, 16 and 17 can transduce signals in the same way. For example, in the figure, layers 15 and 17 both generate green light. In any case, it is the sequence of characteristic signals generated in the sequentially coupled layers that is significant. For example, the characteristic signal produced by multiple layers 15 (green), 16 (red) and 17 (yellow) is Green-Red-Yellow (G-R-Y) in spatial order. This spatial sequence of colors provides a coding scheme that can be used to tag thousands of distinguishable encoders.

During deposition of the multiple layers 15, 16 and 17, heat and/or pressure and/or UV light can be applied to the layers to promote bonding and adhesion between the layers and to laminate or cure (solidify) them. For example, polymeric glass precursors cure into a crystalline glass solid. For some polymeric materials it can be advantageous to deposit additional adhesion promoter materials, such as a curable acrylate, aziridine composites, and epoxides, between layers to promote inter-layer adhesion. Additionally, materials having specific physical properties, such as reflective materials, can be deposited between polymer layers to generate lasing action on the transduced signals.

Depending on the material of the deposited layers, alternative deposition techniques can be employed to generate multiple layers. For example, when the matrix comprises a nanocrystalline or semiconductor material, chemical vapor deposition (CVD) or matrix assisted pulsed-laser deposition (MAPLE) can be a more appropriate technique for sequential deposition of horizontal layers.

After deposition of the multiple layers, photolithography can be used to divide the layers. Photolithography is the process of using light to create a pattern. In the context of the present invention, there are two types: positive and negative. For positive, a mask is exposed with UV light wherever the underlying layers are to be removed. In these layers, exposure to the UV light changes the chemical structure of the layers so that the layers become more soluble in the developer. The exposed layers are then removed, leaving individual microbar encoders. The mask, therefore, contains an exact copy of the pattern of microbar encoders that are to remain. Negative photolithography occurs in just the opposite manner.

For example, after deposition of the multiple polymeric layers 15, 16 and 17 in step S4, a top etching mask layer 20 can be deposited over topmost layer 17. The mask layer is not continuous and only covers and protects sections of layer 17. In one embodiment, the mask layer is produced by first depositing a photoresist layer, curing portions of the photoresist by exposure to ultraviolet light according to a precise negative pattern, then removing uncured portions of the photoresist and re-exposing areas of the topmost layer 17. A metallic layer including titanium is deposited on both the photoresist mask and the exposed sections of layer 17. Finally, the photoresist sections are removed from the topmost layer by severing bonds between the photoresist and the layer 17 using an acetone solution. A pattern of titanium deposits on the top layer 17 remains. This titanium layer then acts as a protective mask during subsequent etching/removal operations in the well-known technique "lift of."

Alternatively, the masking material for protecting the underlying layers during etching can be a metal, which can consist of gold and titanium, where the gold is used to bind organic or hybrid thiol molecules. The thiol molecules then act as a linking agent to bind thiolated oligonucleotide probe molecules, e.g. 50a-d, creating an oligo-sensitive sensor (each of the probe molecules in this case having the same composition).

Another approach consists of depositing a layer of the material that will later become the "stop-mask" first, e.g., titanium. Patterning of the mask is accomplished by depositing a resist layer on top of the mask, and then exposing the wafer to UV light through a photomask containing regions that are opaque and transparent to the UV light. The resist is then "developed" by dipping the wafer in a suitable developer solution. If the resist is "positive," the developer will remove it from the areas that were exposed, i.e., where the photomask was transparent, and if the resist is "negative," the developer will remove the resist from the unexposed areas. In either case, the desired outcome is to expose the stop-mask film (titanium) to the environment in the regions that will be removed by etching, while maintaining covered with resist the regions that will not be removed by etching. The regions of the stop-mask exposed to the environment are then removed by a suitable etchant, for example, NaOH. After the stop-mask has been patterned, the remaining top layer resist regions are removed with a suitable solvent, such as acetone, leaving behind a pattern of separated regions, each covered by a layer of the "stop-layer" material. Subsequently, the wafer is exposed to an anisotropic etch, such as oxygen plasma, which removes the material of the previous layers from the areas not protected by the stop-mask. Thus a plurality of separate columns are created on the substrate. The final stage is to lift off these columns by etching selectively the first layer and freeing the encoded columns.

In another alternative, prior to separating the microbars from the substrate during fabrication, a top plug can be imprinted on top of the microbars by chemical vapor deposition. This plug can comprise silica, a curable sol-gel, a polymer siloxane group, etc. As is known in the art, each of these materials can act as a suitable platform for binding directly to a probe molecule, or for binding to a linking agent that binds to a probe molecule.

In step S5, the microbar encoders are detached from the substrate and columns of microbar encoders are generated by removing the unprotected material leaving standing columns of the polymeric material, e.g., 25a-d from the deposited layers 15, 16 and 17. To remove the material, dry etching techniques such as plasma etching can be employed. When dry etching techniques are employed, any mask layer 20 protects sections, e.g., 30a-d from exposure to the etching gas, while unprotected sections 25a-d are removed. However, other material removal techniques can be employed that cut narrow sections of material directly and thus do not require use of a mask layer to protect sections of the deposited layers. Among such removal techniques are ion milling, laser ablation, and mechanical techniques including dicing and punching. When mechanical techniques are used, care is taken that the dicing and punching surfaces have horizontal dimensions at least as small as, and preferably slightly smaller than, those of the removed sections.

At this point, bars such as 30a-d remain attached to the substrate 10 via the removable layer 12. Separate microbar encoders having substantially identical transducing layered segments are then created by dissolving removable layer 12 using an appropriate solvent, freeing the bars from the substrate 10. For example, when the removable layer 12 consists of aluminum oxide, a potassium hydroxide solution can be applied as a solvent. The microbar encoders can also be freed from the substrate by removing the removable layer 12 from the substrate.

FIGS 1A-C schematically illustrate the microbar encoders produced by the present inventive methods. As shown in FIG 1A, probe molecules 50a-f are bound to the segment 45, which coats the top of the microbar encoder 40. As shown in FIG 1B, probe molecules 70a-i are bound to the outer surface of the microbar encoder, which is composed of layers 62 and 64 and the top coating 66. As shown in FIG 1C, probe molecules 85a-b are bound to the bottom layer 82 and probe molecules 87a-g, which are different than probe molecules 85a-b, are bound to layer 84 of the microbar encoder. Finally, in FIG 1D, probe molecules 90a-i are bound to both the coat layer 100 and the single layer of the microbar encoder 102.

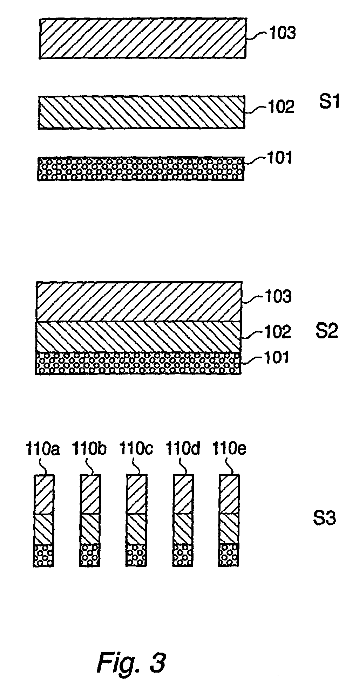

FIG 3 illustrates a second exemplary embodiment of a method of fabricating microbar encoders according to the present invention. In steps S1-3, single layers containing indicator materials are deposited (and/or patterned) and cured in parallel according to the deposition and curing techniques described above. As noted previously, these layers are set down unsupported by a template. The pre-cured layers 101, 102 and 103 can be easily manipulated and are, in step S1, positioned in a multilayer stack. In step S2, the layers are laminated by application of pressure and heat, so that the layers adhere to each other. Then, in step S3, sections of the multilayer stack are removed according to one or more of the removal techniques described above, to create separate microbar encoders 110a-e. In this embodiment there is no need to detach the microbar encoders from an underlying substrate.

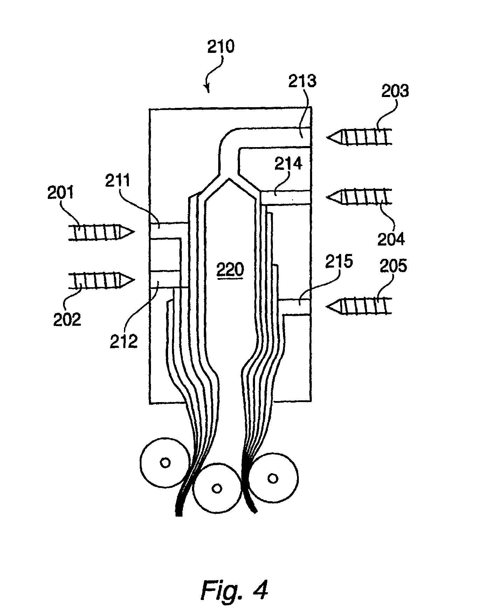

FIG 4 schematically illustrates a coextrusion apparatus. According to another exemplary embodiment of a method of fabrication microbar encoders according to the present invention without use of a template, sheets of polymeric or polymeric precursor material can be converted directly into multilayers. As shown, multiple extruders 201, 202, 203, 204, 205 extrude a polymer matrix comprising or including transducing material into various channels 211, 212, 213, 214, 215 of a coextrusion apparatus 210. The channels are configured so that the various extruded polymer matrices meet and form distinct layers 231, 232, 233, 234, 235 against a barrier 220 situated in the middle of the apparatus. The layered polymer matrices flow together towards a stretching means such as rollers 241, 242, and 243, which stretch the layers into thin multilayered sheets.

Alternatively, a single sheet of freestanding film can be used to manufacture the microbar encoders. The freestanding film can be produced by blending the tags with a thermoplastic material, which is then extruded as a film, or in a polymeric solution that is coagulated into a film, or in a crosslinkable resin that is poured onto a drum and then crosslinked to form a film.

One particularly efficient technique for fabricating single-layer microbar encoders employs a photoresist. In this example, shown in FIG 1, SU-8 (11) (MicroChem Inc., Newton, MA), which is a negative tone photoresist having a high aspect ratio of approximately 20, (see U.S. Patent No. 4,882,245 (Gelorme et al.)), having a specific mixture of transducing material is deposited over a removable or etchable layer 12 on a solid support 10. When deposited, the photoresist layer 15 can be up to 2 mm thick and is soft baked (at approximately 90° C). Additional layers 16-17 of SU-8, having additional mixtures of transducing materials, can be deposited. The layers are then developed with ultraviolet radiation using a photomask 20. Due to the high aspect ratio of the photoresist, the development process forms thin walls of photoresist material (25a-d). These wall then become the microbar encoders 30a-d after detachment from the removable layer, which detachment can be accomplished according to the techniques described above.

To develop microbar encoders linked to multiple probes using the present inventive methods during spin-cast deposition, two composites are blended for each layer; one having a polymer complex bound to a probe molecule, and the other being soluble or degradable in a second solvent. After the microbars are separated, they are bathed in a second solvent to remove the soluble or degraded material, leaving a porous polymer matrix including the probe molecules. The transducing indicator materials can then be linked to the specific probe molecules at each segment of the microbars. As a result, each segment of the microbar encoder is then associated with a characteristic probe molecule and specific transducing materials. FIG 1C schematically illustrates a microbar encoder in which various segments are bound to different probe molecule species. As shown, probe molecules 85a, 85b bound to the bottom segment 82 are of one species (shown in dark color), while the probe molecules 87a, 87b, 87c, 87d and 87e (shown in light color) bound to segment 84 are of another species.

The above methods have described how to fabricate, without a template, a group or set of substantially identical microbar sensors, i.e., microbar encoders plus the probe molecules bound to them, each of which binds to the same target molecule species. However, since the number of layers deposited, the size of the layers and the transducing materials included within each can be varied during separate fabrication operations, a plurality of distinguishable groups of microbar sensors in which each group has a characteristic detectable signal and binds to a specific target molecule, can be produced. Therefore, to provide for massively parallel screening, thousands of distinguishable groups of microbar sensors can be blended together in solution and then this anisotropic assembly can be used to screen for large numbers of target molecules in a sample.

An assembly or collection of microbar encoders or microbar sensors, in the context of the present invention, means a plurality of microbar encoders or sensors that are differentiable from each other. It is not intended to indicate, however, that the members of the assembly are ordered or organized in any particular manner. In such assemblies, at least one of the members of the assembly is linked to a different probe than that of another member, which can detect a different target molecule than that of the other member. The assemblies of the present invention can include from about 2 to about 10¹⁰ different members.

In the foregoing description, the invention has been described with reference to a number of exemplary embodiments that are not to be considered limiting. Rather, it is to be understood and expected that variations in the principles of the method and system herein disclosed may be made by one skilled in the art and it is intended that such modifications, changes, and/or substitutions are to be included within the scope of the present invention as set forth in the appended claims. Detailed descriptions of conventional methods relating to the DNA, RNA and protein analyses discussed herein can be obtained from numerous publication, including Sambrook, J. et al., (1989) Molecular Cloning: A Laboratory Manual, 2^{nd} ed., Cold Spring Harbor Laboratory Press. All references mentioned herein are incorporated by reference in their entirety.

## Claims

1. A method of making a plurality of microbar encoders (40), the microbar encoders having a characteristic detectable signal and capable of linking to a probe molecule, comprising:
(a) depositing one or more layers (15, 16, 17) unsupported by a template, each layer comprising a transducing material, and
(b) dividing the deposited layers into the plurality of microbar encoders (30a-30d), wherein the plurality of microbar encoders have substantially identical characteristic detectable signals.

2. A method as claimed in claim 1, wherein the transducing material is a quantum dot, an organic dye, an inorganic phosphor, a metal-organic phosphor, a fluorescent dye, a pigment, a scattering or absorbing powder, a three-dimensional photoluminescent dendrimer molecule, or any combination thereof.

3. A method as claimed in claim 1 or 2, wherein the probe molecule is capable of binding with a target molecule.

4. A method as claimed in any preceding claim wherein the probe molecule or the target molecule comprises a biological molecule.

5. A method as claimed in any preceding claim wherein one or more of the deposited layers comprises a polymeric matrix.

6. A method as claimed in any preceding claim wherein the deposited layers are divided by dicing or laser ablation; mechanical punching; photolithography; or depositing a patterned mask layer over a surface of the deposited layers, the mask layer protecting a portion of the surface of the deposited layers, and etching through an unprotected portion of the surface of the deposited layers.

7. A method of making a plurality of microbar sensors comprising:
(a) making a plurality of microbar encoders by means of a method as claimed in any preceding claim and
(b) linking a probe molecule to the plurality of microbar encoders.

8. A method of making an assembly of microbar encoders comprising:
(a) making a first plurality of microbar encoders by means of a method as claimed in any of claims 1-7 and
(b) making a second plurality of microbar encoders by means of a method as claimed in any of claims 1-7,
wherein the first and second plurality of microbar encoders have different characteristic detectable signals.

9. A method of making an assembly of microbar sensors comprising:
(a) making a first plurality of microbar sensors by means of a method as claimed in claim 7 and
(b) making a second plurality of microbar sensors by means of a method as claimed in claim 7,
wherein the first and second plurality of microbar sensors have different characteristic detectable signals.

10. A microbar encoder obtainable by means of a method as claimed in any of claims 1-6, wherein only one layer is deposited.
